# EUROPÄISCHE PATENTSCHRIFT

(11) **EP 0 806 972 B1**
(45) Veröffentlichungstag und Bekanntmachung des Hinweises auf die Patenterteilung: **21.11.2001**
(21) Anmeldenummer: 96902961.0
(22) Anmeldetag: 31.01.1996
(51) Int. Cl.: A61M 5/48, A61M 5/315

(54) **VERFAHREN UND VORRICHTUNG ZUM DRUCKKONTROLLIERTEN HANDHABEN EINES FLUIDES, INSBESONDERE FÜR MEDIZINISCHE ANWENDUNGEN DER VORRICHTUNG**
PROCESS AND DEVICE FOR PRESSURE CONTROLLED MANIPULATION OF A FLUID, IN PARTICULAR FOR MEDICINAL APPLICATIONS OF THE DEVICE
PROCEDE ET DISPOSITIF DE MANIPULATION SOUS PRESSION REGULEE D'UN FLUIDE, EN PARTICULIER POUR APPLICATIONS MEDICALES DUDIT DISPOSITIF

(30) Priorität: 02.02.1995 DE 19503230
(43) Veröffentlichungstag der Anmeldung: 19.11.1997
(73) Patentinhaber: Enk, Dietmar, 48653 Coesfeld (DE)
(72) Erfinder: Enk, Dietmar, 48653 Coesfeld (DE)
(74) Vertreter: Kahlhöfer, Hermann, Dipl.-Phys.
(86) Internationale Anmeldenummer: EP9600402
(87) Internationale Veröffentlichungsnummer: WO9623539

(56) Entgegenhaltungen:
- DE-A- 2 714 818
- US-A- 3 998 223
- US-A- 4 624 659

## Beschreibung

Die Erfindung betrifft ein Verfahren und eine Vorrichtung zum druckkontrollierbaren Handhaben eines Fluides. Die Vorrichtung ermöglicht insbesondere eine vergleichmäßigte und kontrollierte, insbesondere druckkontrollierte Injektion eines Fluides, welches eine Flüssigkeit oder ein Gas sein kann. Auch kann die Vorrichtung auf verschiedene Weise als Sonde zum Einführen von Kanülen und beim Setzen von Kathetern eingesetzt werden. Insbesondere betrifft die Erfindung die Weiterbildung einer Spritze für medizinische Zwecke. Bevorzugtes Anwendungsgebiet ist die Anästhesie.

Bekannte Vorrichtungen zum Handhaben eines Fluides, insbesondere Spritzen, weisen einen Behälter zur Aufnahme und Abgabe eines Fluides mit einem in den Behälter einschiebbaren Kolben auf, der zur Innenwand des Behälters durch Dichtmittel abgedichtet ist. Sie werden zumeist dort eingesetzt, wo ein Fluid für eine gewisse Zeit bevorratet wird, bevor man es einsetzt. Außer um medizinische Anwendungen kann es sich dabei um eine Vorrichtung zur Schmierung von Bauteilen, aber ebenso auch um eine Vorrichtung zum genauen druckkontrollierten Dosieren eines Fluides handeln. Bei derartigen Anwendungen sind neben der Dosiermenge der Druck des Fluides bei seiner Injektion, seine Druckänderung oder auch die Geschwindigkeit der Fluidinjektion von Bedeutung. Diese Parameter müssen oftmals eingestellt und kontrolliert werden, um beispielsweise Schäden bei der Injektion des Fluides zu vermeiden.

Im folgenden soll anhand einer Spritze die Funktion und der Einsatz einer bekannten Vorrichtung am Beispiel der Spinalanästhesie erläutert werden.

Ein bei Spinalanästhesien häufig zu beobachtender, in der Regel rasch einsetzender Blutdruckabfall mit reflektorischer Tachykardie - aufgrund einer Blutgefäßerweiterung infolge der Blockade von präganglionären sympathischen Nervenfasern (= Sympathikolyse) - ist eine unerwünschte, im Einzelfall auch nicht ungefährliche Komplikation dieses Anästhesieverfahrens. Eine besser zu steuernde und in ihrer Ausbreitung kalkulierbare Spinalanästhesie und eine dadurch erreichbare bessere Kreislaufstabilität gerade bei herz-kreislaufkranken Patienten ist daher Wunsch jedes Regionalanästhesisten.

Die alleinige Verwendung sogenannter hyperbarer Lokalanästhetika, die durch Zusatz von Glukose schwerer als der Liquor sind und sich somit im Liquorraum im Gegensatz zu den isobaren Lokalanästbetika vorzugsweise in die tief liegenden Bereiche verteilen, führt in der klinischen Praxis nicht - wie zu erwarten - zu einer besseren Steuerbarkeit einer Spinalanästhesie.

Eigene in-vitro-Untersuchungen haben gezeigt, daß die heute üblichen Injektionstechniken bei Verwendung dünner Spinalnadeln eine erhebliche Verwirbelung des Lokalanästhetikums im Liquorraum mit der Folge einer vollständigen oder zumindest weitgehenden Durchmischung auch hyperbarer Lokalanästhetika mit dem Liquor bedingen. Diese Injektionstechniken sind nicht geeignet, den Liquor verwirbelungsarm bzw. -frei mit einem hyperbaren Lokalanästhetikum zu unterschichten und so eine notwendige Voraussetzung zu schaffen, um durch geeignete Lagerungsmanöver des Patienten die Ausbreitung der Anästhesie zu beeinflussen. Durch den bei hohen Injektionsgeschwindigkeiten resultierenden Injektionsstrahl sind darüberhinaus auch Nervenirritationen oder sogar -traumatisierungen vorstellbar.

Insbesondere eine langsame und gleichmäßige Injektion kann bei Verwendung dünner Spinalnadeln, bei denen es im Vergleich zu dickeren Spinalnadeln bei gleicher Injektionsgeschwindigkeit schneller zu einem turbulenten Injektionsstrahl kommt, Verwirbelungen des Lokalanästhetikums im Liquor und mögliche Nervenirritationen bzw.-traumatisierungen durch den Injektionsstrahl vermeiden. Bei Verwendung dünner Spinalnadeln ist eine solche langsame und gleichmäßige Injektion mit einer konventionellen Spritze von Hand nicht zu leisten, da aus minimalen Lastwechseln auf dem Spritzenkolben (= Änderungen des durch den Finger einwirkenden Druckes) Ungleichmäßigkeiten des Injektionsstrahles resultieren, die zu Verwirbelungen des Lokalanästhetikums im Liquor führen.

Bekannt ist, daß sich bei einer langsamen Injektion eine gewisse Vergleichmäßigung der Injektionsgeschwindigkeit durch eine Luftdämpfung erreichen läßt. Hierzu werden in eine Spritze zusätzlich zum Lokalanästhetikum einige Milliliter Luft aufgezogen; mittels eines Drei-Wege-Hahnes (als 90-Grad-Umlenkung) wird die Spritze senkrecht an die Spinalnadel gesetzt, so daß sich die Luft bei der Injektion zwischen dem Lokalanästhetikum und dem Kolben befindet. Der Kolben wird nachfolgend gerade immer soweit eingeschoben, daß man ein langsames Sinken des Lokalanästhetika-Spiegels in der Spritze beobachten kann.

Aufgrund einer nur wenige Millimeter messenden Distanz zwischen den Nervenfasern der linken zu den Nervenfasern der rechten Körperhälfte ist eine "echte" d.h. motorisch, sensibel und vegetativ (insbesondere sympathisch) einseitige Spinalanästhesie die klinische Anwendung, an der die Leistungsfähigkeit einer bestimmten Technik bzw. bestimmter Vorrichtungen und Hilfsmittel hinsichtlich der damit erreichbaren Steuerbarkeit und Kalkulierbarkeit einer Spinalanästhesie untersucht werden kann. Ein Verfahren, das die Anlage einer "echten" einseitigen Spinalanästhesie ermöglicht, ist auch geeignet, die Steuerbarkeit und somit die Sicherheit bei der konventionellen, beidseitigen Spinalanästhesie zu erhöhen.

Eine eigene klinische Studie zeigt, daß durch eine, wie oben geschildert, luftgedämpfte, langsame und gleichmäßige Injektion (ca. 0,5 ml/min) eines hyperbaren Lokalanästhetikums unter Verwendung einer dünnen Spinalnadel mit seitlich liegender Austrittsöffnung (27 G Whitacre) bei einem seitlich liegenden Patienten motorisch und sensibel strikt oder zumindest weitgehend einseitige Blockaden möglich sind, dabei unerwünschte Sympathikolyse-Effekte auf der nicht zu betäubenden Seite minimiert oder sogar gänzlich vermieden werden können und das Niveau der Spinalanästhesie durch Lagerung des Patienten besser als bislang steuerbar wird.

Der Zeitbedarf für eine solche Injektion ist größer, aber durch kürzere Anschlagzeiten hinsichtlich der Ausbildung der sensiblen und motorischen Blockade läßt sich in der Summe Zeit gewinnen. Die bisherigen Ergebnisse machen es vorstellbar, daß durch ein gezieltes Einbringen des Lokalanästhetikums in den Liquorraum die notwendige Lokalanästhetika-Menge reduziert werden kann (geringeres Volumen bzw. niedrigere Konzentration). Diese Injektionstechnik scheint auch bei einem Glukosezusatz niedrigerer Konzentration als bislang in hyperbaren Lokalanästhetika üblich noch eine sichere Unterschichtung des Liquors mit dem Lokalanästhetikum zu erlauben, so daß ein Arbeiten mit einem hyperbaren aber isoosmolaren Lokalanästhetikum möglich wird. Unerwünschte Hyperosmolaritätseffekte an den Nervenfasern ließen sich so ausschließen.

Die geschilderte Behelfslösung für eine luftgedämpfte Injektion erweist sich zwar als tauglich, nötigt aber zu einem unergonomischen, umständlichen Arbeiten mit einer unhandlichen Konstruktion aus Spinalnadel, Drei-Wege-Hahn und Spritze. Von daher ist eine Vorrichtung, insbesondere eine Spritze wünschenswert, die ergonomisch und in gewohnter Weise zu handhaben ist und bei sicherer Kontrolle über den Injektionsdruck eine langsame und gleichmäßige beziehungsweise vergleichmäßigte Injektion des Lokalanästhetikums ermöglicht.

Heute vorhandene Spritzen mit in bzw. an den Kolben ein- bzw. angebrachten Gummi- oder Federelementen (DE 27 14 818 A1, DE 32 00 651 A1) vermögen den Injektionsdruck mehr oder weniger genau anzuzeigen, schaffen aber nur sehr bedingt eine, z.B. im Rahmen einer Spinalanästhesie, gewünschte Vergleichmäßigung des Injektionsdruckes insbesondere kleiner, kurzfristig auf den Kolben der Spritze einwirkender Druckänderungen. Der teilweise komplexe Aufbau solcher Spritzen und eine notwendigerweise präzise und somit teuere Fertigung schließen den Einsatz solcher Spritzen als sterile Einmalartikel - wie bei bestimmten Anwendungen, z.B. der Spinalanästhesie, heute als Hygiene-Standard gefordert - nahezu aus.

Eine Spritze mit Kolbendruckdämpfung (siehe nicht vorveröffentlichte deutsche Patentanmeldung P 44 28 467.5) leistet, wie eigene in-vitro-Untersuchungen gezeigt haben, eine Vergleichmäßigung des Injektionsdruckes und ermöglicht langsame und gleichmäßige Injektionen einer Flüssigkeit (z.B. Lokalanästhetikum) aus freier Hand. Durch zwei im Spritzzylinder hintereinander geschaltete, voneinander durch Luft vollständig getrennte Kolben wirkt sich der auf dem hinteren Kolben (= Kompressionskolben) lastende Druck des Fingers durch die zwischen den Kolben befindliche komprimierte Luft immer gedämpft auf den vorderen Kolben (= Injektionskolben) aus. Durch Volumenveränderung der zwischen den Kolben befindlichen Luft und Nutzung der Länge des vorderen Kolbens als indirektes Maß für die maximal zulässige und aufrechtzuerhaltende Kompression - unter Vermeidung des direkten Kontaktes der beiden Kolben - kann die gewünschte Injektionsgeschwindigkeit eingestellt und gehalten werden. Die präzise Funktion dieser Spritze setzt einen über den gesamten Verschiebeweg der Kolben exakt maßhaltigen Spritzzylinder und für eine bestimmte Injektionsgeschwindigkeit ein bestimmtes Luftvolumen zwischen den Kolben voraus. Diese relativ hohen Fertigungsanforderungen bedingen einen, verglichen mit in Massenproduktion gefertigten low-cost-Einwegspritzen, deutlich höheren Preis einer solchen Spritze mit Kolbendruckdämpfung.

Die momentan verfügbare Lösung für das Problem einer langsamen und gleichmäßigen Injektion ist eine auch bei kleinen Förderraten präzise laufende Injektionspumpe. Solche Pumpen sind technisch ebenfalls sehr aufwendig, dadurch in Anschaffung und Wartung teuer und - bezogen auf die Situation bei einer Spinalanästhesie - in der Handhabung zu umständlich und zu zeitaufwendig.

Aus der U.S. 4,624,659 wiederum ist eine Spritze für ein geschlossenes Drucksystem, beispielsweise in Verbindung mit einem Ballonkatheter, bekannt. Die Spritze weist einen Kanal auf, der sich durch einen Spritzkolben der Spritze erstreckt. Das eine Ende des Kanals mündet in einen Boden des Spritzkolbens, während das andere Ende des Kanals mit einer elastomeren Membran abgedeckt ist. Über Druckausübung auf den Spritzkolben wird nun ein am Katheder angeordneter Ballon aufgeblasen. Die Membran ist so flexibel, daß sie sich je nach im Ballon anliegendem Druck entweder in den Kanal einstülpt oder aber vom Kanal weg ausstülpt. Um die Größe des jeweilig anliegenden Druckes abschätzen zu können, ist die Membran von einem auf dem Spritzkolben aufsitzenden Indikatorring umgeben, in dessen Mitte ein Freiraum zur Ausstülpung der Membran vorhanden ist. Auf diesen Indikatorring drückt nun der Bediener. In dem Moment, in dem der Druck im Ballon so groß ist, daß dieser eine erwünschte Größe beispielsweise innerhalb einer Ader angenommen hat, gerät die sich ausstülpende Membran in Kontakt mit dem auf den Spritzkolben drückenden Finger des Bedieners. Auf diese Weise wird letzterem angezeigt, daß der gewünschte Zustand des Ballons erzielt ist. Das Material der Membran muß dazu ein der gewünschten Größe des Ballons bzw. dem erwünschten Druck im Ballon entsprechendes elastisches Verhalten haben.

Aufgabe der Erfindung ist die Schaffung eines Verfahrens und einer Vorrichtung mit der ein Fluid genauer druckkontrollierbar gehandhabt werden kann, wobei die Vorrichtung insbesondere bei Injektionen und anderen medizinischen Maßnahmen am menschlichen Körper einsetzbar ist.

Diese Aufgabe wird mit einem Verfahren mit den Merkmalen des Anspruches 1 bzw. mit einer Vorrichtung mit den Merkmalen des Anspruches 5 gelöst. Vorteilhafte Ausgestaltungen und Weiterbildungen der Erfindung ergeben sich aus den jeweils abhängigen Ansprüchen oder durch Kombinationen der Merkmale aus diesen Ansprüchen.

Die Erfindung schafft ein Verfahren zur druckkontrollierbaren Handhabung eines Fluids, ausgenommen Verfahren zur chirurgischen oder therapeutischen Behandlung des menschlichen oder tierischen Körpers und Diagnostizierverfahren, die am menschlichen oder tierischen Körper vorgenommen werden, wobei
- das in einem Behälter von variablem Volumen befindliche Fluid durch einen auf das Fluid einwirkenden Druck an einem Austritt mit diesem Druck zur Verfügung gestellt wird,
- der Behälter mit einem Kanal von vorgegebenem Querschnitt in Verbindung steht,
- der Kanal mit einem Puffer kommuniziert, wobei der Puffer ein Volumen hat,
- der Meßkanal und der Puffer mit einem kompressiblen Medium gefüllt sind, dadurch gekennzeichnet,
daß das Fluid entsprechend dem einwirkenden Druck, dem Querschnitt des einen Meßkanal bildenden Kanals und der vorgegebenen Größe des Volumens des Puffers zumindest teilweise in den Meßkanal eindringt, wobei während de Handhabung des Fluides die Position der Grenzschicht zwischen dem Fluid und dem kompressiblen Medium in dem Meßkanal beobachtet oder gemessen wird und das Handhaben das Erzeugen eines gewissen Druckes in dem Behälter (1a) und das Bewegen einer mit dem Auslaß (1c) verbundenen Kanüle (5b) in einen Körper (9) umfaßt, um ein Gebiet (10) in dem Körper (9) aufzufinden und zu identifizieren, welches einen von dem erzeugten gewissen Druck abweichenden Druck aufweist, indem die Position der Grenzschicht (8) beobachtet wird.
Entsprechend wird auch eine Vorrichtung geschaffen zum druckkontrollierbaren Handhaben eines Fluids, mit
- einem Behälter von variablem Volumen zur Aufnahme eines Fluids,
- einer Druckerzeugungseinrichtung zur Erzeugung eines auf das Fluid einwirkenden Druckes,
- einem Austritt zum Einlaß und/oder Auslaß des Fluides,
- einem Kanal von vorgegebenem Querschnitt, der mit dem Behälter in Verbindung steht, dadurch gekennzeichnet, daß die Vorrichtung einen Puffer von vorgegebenem Volumen hat, der mit dem Kanal als Meßkanal kommuniziert und der zum Zeitpunkt des druckkontrollierten Handhabens bis auf eine mögliche Höhe eines Fluides im Querschitt des Meßkanals durch feste Wände beschränkt wird,
wobei der Meßkanal und der Puffer mit einem kompressiblen Medium gefüllt sind.

Nachfolgend sollen die Erfindung anhand der Funktionsweise einer erfindungsgemäßen Spritze und verschiedene Verfahren für das Arbeiten mit dieser Spritze erläutert werden. Die Erfindung ist jedoch nicht nur auf diese bevorzugten Ausführungsformen und die dargestellten Anwendungsbereiche beschränkt, sondern kann auf andere, vergleichbare, insbesondere auch nichtmedizinische Bereiche mit gleich- oder ähnlich gelagerten Problemen angewendet werden.

Die Vorrichtung ist eine Spritze für die Spinalanästhesie, mit der ein Lokalanästhetikum, insbesondere durch dünne Spinalnadeln, ohne zusätzlichen technischen oder personellen Aufwand von Hand langsam und gleichmäßig in den Liquor injiziert werden kann, um so Verwirbelungen des Lokalanästhetikums mit der daraus resultierenden unerwünschten und unkalkulierbaren Ausbreitung der Spinalanästhesie und Traumatisierungen von Nervenfasern durch den Injektionsstrahl zu vermeiden. Die Spritze ist produktionstechnisch einfach und auch auf schnell laufenden Automaten mit ausreichender Präzision in Massenproduktion kostengünstig herstellbar.

Die Spritze weist in einer vorteilhaften Ausführungsform eine Dämpfungs- und Druckmeßeinrichtung auf, welche einen im Kolben der Spritze liegenden Puffer (mit einem kompressiblen Medium gefüllten Hohlraum) mit festem oder variablem Volumen und einen von der im Spritzenzylinder befindlichen Seite des Kolbens in diesen Puffer führenden, zentrisch oder exzentrisch im Puffer liegenden, zu beiden Seiten hin offenen Meßkanal enthält. Der Puffer und der Meßkanal sind mit einem kompressiblen Medium, insbesondere Luft unter Umgebungsdruck, gefüllt.

Durch Zurückziehen des Kolbens wird der durch Außenwand der Sritze und Boden des Kolbens gebildete Behälter vergrößert und es entsteht im Spritzenzylinder ein Unterdruck, durch den eine Flüssigkeit, z.B. ein Lokalanästhetikum, genauso schnell wie bei einer konventionellen Spritze in den Spritzenzylinder aufgezogen werden kann.

Beim Entlüften der beispielsweise senkrecht gehaltenen Spritze verschließt nun die in den Spritzenzylinder aufgezogene Flüssigkeit die vordere, der Flüssigkeit zugewandte erste Öffnung des in den Puffer des Kolbens führenden Meßkanales. Diese Öffnung des Meßkanales darf dabei, abhängig von der Viskosität der Flüssigkeit, einen bestimmten Durchmesser nicht überschreiten, da beim Entlüften der Spritze die Flüssigkeit sonst in den Meßkanal laufen kann. Bei einem nicht zu großen Durchmesser von z.B. nicht mehr als 1 bis 3 mm² der vorderen Öffnung des Meßkanales reicht die Oberflächenspannung der Flüssigkeit für den Verschluß. Zusätzliche Trennungsmittel, z.B. ein elastischer Stopfen oder ein Tropfen Öl, werden aber bei einer Injektion eines Gases gemäß einer vorteilhaften Weiterentwicklung der Spritze verwendet. Die Trennungsmittel sind dabei so auszuwählen, daß der Dämpfungseffekt und die Druckmeßgenauigkeit sich nur wenig ändern. Sie sollten so leicht im Meßkanal verschiebbar sein, daß ihre Reibung gegenüber den sonst auftretenden Kräften vernachlässigbar ist.

Durch Abschluß der im Meßkanal und Puffer des Kolbens befindlichen Luft entsteht bei dieser Spritze gleichzeitig ein Dämpfungselement und eine Druckmeßeinrichtung im Kolben.

Überschreitet nun beim Einschieben des Kolbens in den Spritzenzylinder, d. h. beim Verkleinern des Behälters, der Druck, mit dem die Flüssigkeit durch eine kleinlumige Kanüle mit relativ hohem Durchflußwiderstand gespritzt wird, einen bestimmten Betrag, so wird die Flüssigkeit teilweise in den Meßkanal - aufgrund des hier vergleichsweise niedrigeren Durchflußwiderstandes - gedrückt. Dadurch kommt es zu einer Verdrängung der im Meßkanal befindlichen Luft und somit einer Kompression der im Meßkanal und Puffer befindlichen Luft. Wird nun nachfolgend dieser Druck, durch den ein bestimmter Kompressionsgrad der Luft erreicht wurde, unterschritten, so wird die in den Meßkanal eingedrungene Flüssigkeit unter Dekompression der komprimierten Luft im Meßkanal und Puffer wieder aus dem Meßkanal herausgedrückt.

Einerseits werden so plötzliche Änderungen des auf den Kolben und somit auf die Flüssigkeit einwirkenden Druckes - wie bei einer Injektion von Hand bei zu schnellem Einschieben des Kolbens in den Spritzenzylinder nicht zu vermeiden - wirkungsvoll gedämpft. Dabei wird durch den direkten, kraftschlüssigen Kontakt der Flüssigkeit zur Luft als dämpfendes, weil kompressibles Medium ein ausgeprägter Dämpfungseffekt erreicht. Andererseits wird der Fluß der Flüssigkeit durch eine auf den Kanülenansatz der Spritze aufgesteckte kleinlumige Kanüle vergleichmäßigt.

Durch geeignete Dimensionierung des Kolbens an der in den Spritzenzylinder eingeschobenen Seite, durch am Kolben angebrachte Dichtmittel, z.B. eine zirkuläre Dichtlippe, oder andere geeignete Mittel wird in einer weiteren günstigen Ausführungsform ein großer Verschiebewiderstand erzielt. Dieser soll einerseits ein weitgehend ruckfreies Einschieben des Kolbens in den Spritzenzylinder gestatten, andererseits muß durch diesen Verschiebewiderstand sichergestellt sein, daß auch bei Entlastung, insbesondere vollständiger Entlastung durch den Finger, der Kolben seine Einschiebtiefe behält und nicht durch die komprimierte Luft im Meßkanal und Puffer im Spritzenzylinder zurückgedrückt wird. Der Einsatz einer derartigen Spritze vermeidet eine zu schnelle Flußverlangsamung der Flüssigkeit durch die Kanüle. Der Verschiebewiderstand ist in einer günstigen Weiterbildung auch so dimensionierbar, daß ein gewisses Spiel der Einschiebtiefe des Kolbens bei Druckentlastung zugelassen wird.

Die für eine langsame und gleichmäßige bzw. vergleichmäßigte Injektion wichtige Dämpfungseigenschaft einer erfindungsgemäßen Spritze wird durch einen relativ hohen Durchflußwiderstand, z.B. einer kleinlumigen Kanüle, gegen den die Flüssigkeit injiziert wird, und durch einen vergleichsweise niedrigen Durchflußwiderstand zumindest eines Teils des Meßkanales begünstigt. Der Meßkanal mündet günstigerweise an seinem einen, dem hinteren Ende in den Puffer des Kolbens. Ein auf den Kanülenansatz des Spritzenzylinders gestecktes Durchflußreduzierstück, das den notwendigen, relativ hohen Durchflußwiderstand schafft, ermöglicht den Einsatz einer erfindungsgemäßen Spritze auch bei großlumigen Kanülen, die einen relativ großen Innendurchmesser und somit einen - für -eine einwandfreie Funktion der Spritze - zu kleinen Durchflußwiderstand bieten.

Während der Injektion einer Flüssigkeit resultiert der Druck, mit dem die Flüssigkeit injiziert wird, bei einer Spritze mindestens aus zwei in ihrem Einzelbetrag, zu verschiedenen Zeitpunkten, variablen, aber in der Summe nahezu gleichbleibenden Teildrücken: Durch das Einschieben des Kolbens in den Spritzenzylinder lastet der Druck des Fingers direkt auf der im Spritzenzylinder befindlichen Flüssigkeit. Andererseits führt ein zu großer direkter Druck, beispielsweise bei einem raschen Einschieben des Kolbens, zu einem Eindringen der Flüssigkeit in den Meßkanal und einer Kompression der im Meßkanal und im Puffer des Kolbens befindlichen Luft. Hier baut sich dadurch ein Druck auf, der erst bei Nachlassen des auf dem Kolben lastenden Druckes des Fingers für die Injektion der Flüssigkeit wirksam werden kann und insofern als indirekter Druck bezeichnet wird. Schiebt man den Kolben sehr schnell in den Spritzenzylinder, erfolgt die Injektion der Flüssigkeit zu diesem Zeitpunkt hauptsächlich durch einen direkten Druck; entlastet hingegen der Finger den Kolben vollständig, wird die im Spritzenzylinder befindliche Flüssigkeit durch einen indirekten Druck - unter Dekompression der im Meßkanal und Puffer des Kolbens vorhandenen komprimierten Luftinjiziert.

Der jeweils auf die Flüssigkeit wirkende Druck kann in einer vorteilhaften Ausführungsform der Erfindung am Meßkanal beobachtet oder gemessen werden (weiter in den Meßkanal laufende Flüssigkeit = zunehmender Druck; zurücklaufende Flüssigkeit = fallender Druck). Mittels einer für einen bestimmten Durchflußwiderstand, z.B. einer kleinlumigen Kanüle, geeichten, beispielsweise auf oder über dem Meßkanal angebrachten Skala kann der Druck aus der Position der Grenzschicht zwischen Flüssigkeit und kompressiblem Medium (Meniskus) als Absolutwert oder direkt die Injektionsgeschwindigkeit - nach Ermittlung der bei Verwendung einer bestimmten Kanüle und bestimmter Flüssigkeitsstände im Meßkanal resultierenden unterschiedlichen Injektionsgeschwindigkeiten - angegeben werden.

Eine gute Ablesbarkeit einer solchen Skalierung ist mittels eines hochtransparenten Werkstoffes, insbesondere Kunststoff, erzielbar. Da bei einem mittig im Puffer angeordneten Meßkanal der Flüssigkeitsstand bei einer klaren Flüssigkeit durch insgesamt drei Kunststoffschichten erkannt werden muß, ist es vorteilhaft, den Meßkanal exzentrisch im Puffer anzuordnen, also an die Wand des Kolbens zu legen. Daraus folgt, daß der Flüssigkeitsstand im Meßkanal vor allem aus einer Richtung gut und sicher beobachtet werden kann. Wählt man für den Meßkanal einen ovalen Querschnitt, so ist der Flüssigkeitsspiegel von der breiteren Seite des Meßkanales betrachtet unter Umständen, z.B. bei geeigneter Lichtbrechung durch die wie eine Lupe wirkende ovale Geometrie, besser erkennbar, ohne daß dadurch das Volumen des Meßkanales zunehmen muß.

Eine günstige Lösung ist es, den Meßkanal in die Wand des Kolbens zu integrieren oder von außen in eine Rinne des Kolbens zu legen, so daß der Flüssigkeitsstand im Meßkanal in diesem Fall nur durch zwei Kunststoffschichten zu beobachten ist. Ein weiterer Vorteil dieser Lösung besteht darin, daß durch Parallaxe bedingte Ablesefehler des Flüssigkeitsstandes an einer von außen auf den Kolben aufgebrachten Skalierung minimiert werden. Diese ist produktionstechnisch einfach und billig herzustellen.

Eine weitere vorteilhafte Ausführungsform der Erfindung sieht zusätzlich im Spritzenzylinder eine längliche Aussparung vor. In diesem Fall ist der Flüssigkeitsstand durch nur eine Kunststoffschicht noch besser zu erkennen.

Neben einer Strich-Skalierung sind auch verschiedenfarbige Ring- oder Balkenmarkierungen (z.B. grün = korrekte Injektionsgeschwindigkeit, gelb = noch korrekte Injektionsgeschwindigkeit, rot = zu schnelle Injektionsgeschwindigkeit) oder auch unterschiedliche Querschnitte des Meßkanales als Kennzeichnung bestimmter Injektionsgeschwindigkeitsbereiche vorstellbar.

Das Einhalten einer bestimmten, beispielsweise maximal zulässigen Injektionsgeschwindigkeit kann dadurch definiert sein, daß der Flüssigkeitsstand im Meßkanal eine bestimmte Marke nicht überschreiten darf oder die Flüssigkeit den Meßkanal zwar vollständig füllen, aber nicht aus dem Meßkanal abtropfen darf. Bei dieser einfacheren Arbeitsvorschrift ist eine korrekte, d.h. nicht zu schnelle Injektion im nachhinein gut zu überprüfen: Ein Tropfen der Flüssigkeit im Puffer zeigt einen Injektionsfehler an.

Eine Messung der bisher genannten und weiterer Parameter ist ebenfalls durch Einsatz von anderen, z.B. induktiven, opto-elektronischen oder piezo-elektrischen Meßeinrichtungen möglich. Die Parameter können dann eingestellt, kontrolliert, gesteuert oder auch geregelt werden, je nachdem, welche Bedingungen mittels der Erfindung eingehalten werden sollen.

Eine schrittweise Erhöhung des auf dem Kolben lastenden Druckes um den jeweils gleichen Betrag hat aufgrund der zunehmenden Kompression der Luft im Meßkanal und Puffer kleiner werdene Veränderungen des Flüssigkeitsstandes im Meßkanal zur Folge: Bei einem kleinen Volumen im Puffer und einem relativ großen Volumen im Meßkanal kann man somit einen relativ großen Druckbereich über eine relativ kurze Skala erfassen; hierbei werden die Druckänderungen bei noch geringer Kompression der Luft im Meßkanal und Puffer empfindlich und mit zunehmender Kompression unempfindlicher angezeigt. Hingegen sind bei einem relativ großen Volumen im Puffer und Auslegung des Meßkanales als lange Kapillare selbst kleine Druckänderungen sehr empfindlich zu erfassen, da diese eine deutliche Änderung des Flüssigkeitsstandes im Meßkanal bewirken.

Eine effektive Dämpfung plötzlicher, durch den Finger auf den Kolben einwirkender, direkter Druckanstiege und ein vergleichmäßigter Fluß der Flüssigkeit durch eine kleinlumige Kanüle mit einer bei kurzfristiger Entlastung des Kolbens durch den Finger nur langsam abnehmenden Injektionsgeschwindigkeit erfordert bei einer erfindungsgemäßen Spritze einen, im Vergleich zum Durchflußwiderstand der an den Kanülenansatz des Spritzenzylinders angesetzten Kanüle, relativ kleinen Durchflußwiderstand eines Teiles des Meßkanales, beispielsweise der vorderen, ersten Meßkanalöffnung, und ein relativ großes Dämpfungsvolumen im Meßkanal und/oder Puffer. Um eine sichere Kontrolle über die Injektionsgeschwindigkeit zu haben, müssen dabei Änderungen des auf der Flüssigkeit lastenden Druckes immer ausreichend deutliche, d.h. gut erkennbare Veränderungen des Flüssigkeitsstandes im Meßkanal ergeben.

Das Verhältnis des Volumens im Meßkanal zu dem im Puffer hat Einfluß auf das Dämpfungsverhalten einer erfindungsgemäßen Vorrichtung, insbesondere einer Spritze. Bei einem relativ großen Volumen im Puffer wird die Luft bei einer bestimmten Zunahme des Flüssigkeitsvolumens im Meßkanal weniger stark komprimiert als bei einem kleinen Volumen im Puffer. Bei Dekompression eines großen, schwach komprimierten Luftvolumens folgt ein langsames und in sich recht gleichmäßiges Zurückdrücken der Flüssigkeit aus dem Meßkanal; bei Dekompression eines kleinen, stark komprimierten Luftvolumens resultiert hingegen ein insbesondere anfänglich schnelleres und sich dann deutlich verlangsamendes Zurückdrücken der Flüssigkeit aus dem Meßkanal. Das Dämpfungsvolumen besitzt somit auch die Funktion eines Puffers.

Bei einer Weiterbildung der erfindungsgemäßen Spritze kann das Luftvolumen im Puffer des Kolbens durch unterschiedliche Verschlußstücke in bestimmten Schritten bzw. durch ein im Kolben bewegliches Verschlußstück in einem bestimmten Rahmen stufenlos variiert werden.

Dadurch kann das Dämpfungsverhalten der Spritze auf einfache Weise verändert werden, die Spritze also "weicher" oder "härter" eingestellt werden. Bezogen auf den gleichen Flüssigkeitsstand im Meßkanal läßt sich so die Injektionsgeschwindigkeit bei gleichem Durchflußwiderstand, z.B. einer bestimmten Kanüle, verändern. Interessant ist auch die Möglichkeit, bei unterschiedlichen Durchflußwiderständen, z.B. durch verschiedene Kanülen, eine gleichbleibende Injektionsgeschwindigkeit einzustellen. Dies minimiert die Produktionskosten: Zum einen behält eine Skalierung, beispielsweise auf bzw. über dem Meßkanal mit Angabe der Injektionsgeschwindigkeit, ihre Gültigkeit; zum anderen produziert man durch Einsatz verschiedener Verschlußstücke preiswert Spritzen bzw. Spritzenkolben mit unterschiedlichsten Puffervolumina für beispielsweise unterschiedliche Kanülen.

Verschließt man durch ein geeignetes Verschlußstück die hintere, zweite Öffnung des Meßkanales, also die Mündung des Meßkanales in den Puffer des Kolbens, so dämpft der Kolben nur noch über das kleine, im Meßkanal befindliche Luftvolumen, somit also "sehr hart" und mißt den Druck entsprechend unempfindlicher. Bei einem sehr kleinen Luftvolumen im Meßkanal hat dies zur Folge, daß sich die Spritze nahezu wie eine konventionelle Spritze verhält. Ist das Luftvolumen im Meßkanal hingegen etwas größer, so wird mit einer solchen Spritze, ohne die Notwendigkeit, einen zusätzlichen Spritzenkolben fertigen zu müssen, ein Arbeiten in zwei verschiedenen Dämpfungs- und Druckmeßbereichen möglich, die durch Verschieben eines geeigneten Verschlußstückes im Kolben gewählt werden können.

Erfindungsgemäß kann in den Kolben einer Spritze ein größerer, insbesondere wesentlich größerer Luftdämpfer integriert werden, ohne die Länge der Spritze, im Vergleich zu einer konventionellen, verändern zu müssen.

Bei Sterilisation der bekannten Spritzen können Bauelemente insbesondere durch wiederholte Hitze-Sterilisation beschädigt werden; die Verwendung von Metallteilen macht eine Sterilisation mit Gamma-Strahlen schwierig. Insbesondere innenliegende, durch entsprechende Dichtungen weitgehend oder vollständig abgeschlossene Lufträume sind bei bestimmten Sterilisationsverfahren (Hitze, Äthylenoxid) problematisch. Hingegen kann eine erfindungsgemäße Spritze - wenn auch durch den verwendeten Kunststoff limitiert - prinzipiell jedem Sterilisationsverfahren unterzogen werden. Vorteilhaft dafür ist, daß das im Kolben befindliche Luftvolumen erst durch Aufziehen der Flüssigkeit in den Spritzenzylinder abgeschlossen wird.

Da bei einer Sterilisation der Spritze die im Meßkanal und Puffer des Kolbens befindliche Luft zwangsläufig auch sterilisiert wird, wirft der direkte Kontakt der Flüssigkeit mit dieser Luft kein hygienisches Problem auf. Im Vergleich zu einer Behelfslösung, bei der unsterile Raumluft verwendet wird, bietet eine Spritze auch in diesem Punkt größtmögliche Sicherheit für den Patienten.

Eine erfindungsgemäße Spritze ermöglicht ein ergonomisches, z.B. für einen Anästhesisten gewohntes und dadurch in der Anwendung am Patienten sicheres Arbeiten. Dies stellt im Vergleich zu -einer unhandlicheren und umständlicheren Behelfslösung sicher, daß bei einer Spinalanästhesie über den Injektionszeitraum die korrekte Tiefenposition der Spinalnadel nicht verlorengeht.

Eine erfindungsgemäße Spritze erfordert im wesentlichen keine Präzisionsteile. Der Kolben kann als einfaches Kunststoffspritzteil gefertigt werden. Die produktionstechnisch einfachste und zudem in der Anwendung flexibelste Lösung ist es, den Kolben einer Spritze als - bis auf die vordere, erste Mündung des Meßkanales - einseitig verschlossenen Zylinder zu fertigen und dessen rückwärtige, offene Seite durch ein geeignetes Verschlußstück, z.B. aus Gummi oder Kunststoff, zu verschließen, so daß ein innenliegender Hohlraum als Puffer entsteht.

Eine zirkuläre, in den Kolben eingesetzte Dichtlippe, z.B. aus Gummi, schafft auch in einem, vom Durchmesser her nicht exakt maßhaltigen Spritzenzylinder einen ausreichend gleichmäßigen Verschiebewiderstand und verhindert ruckartige Bewegungen des Kolbens beim Einschieben. Eine einfache und kostengünstige Lösung ist es, bei der Fertigung des Kolbens eine solche zirkuläre Dichtlippe direkt anzuspritzen, wie bei den Kolben sogenannter low-cost-Einwegspritzen üblich. Eine gute Dämpfung der möglichen abrupten Bewegungen eines solchen Kolbens beim Einschieben im Spritzenzylinder erfordert aber eine ausreichend große Auslegung des Dämpfungsvolumens im Meßkanal und Puffer.

Eine erfindungsgemäße Spritze kann auf schnell laufenden Automaten gefertigt werden. Das Problem der Wiederholgenauigkeit des zwischen den beiden Kolben einer Spritze mit Kolbendruckdämpfung eingeschlossenen Luftvolumens stellt sich nicht, da das Luftvolumen im Meßkanal und Puffer des Kolbens nur im Bereich der Fertigungsungenauigkeit der beispielsweise für den Kolben benötigten Teile insbesondere bei Fertigung aus Kunstoff minimal und nicht relevant schwankt.

Diese Spritze ermöglicht aber nicht nur eine langsame und vergleichmäßigte Injektion eines Lokalanästhetikums bei der Spinalanästhesie, sondern kann auch zur druckkontrollierten Injektion anderer Flüssigkeiten verwendet werden, z.B. beim "Blocken" eines transurethralen Katheters. Dabei wird mit Flüssigkeit ("physiologische" Kochsalzlösung) ein Ballon an der in der Blase liegenden Spitze des Katheters gefüllt. Kommt es nun, mittels der Spritze gut beobachtbar, zu einer ungewohnt hohen Drucksteigerung während der Katheter "geblockt" wird, ist dies ein Anhalt für eine Fehllage des Katheters. Sonst zu spät bemerkte Katheterfehllagen können so, gerade beim narkotisierten Patienten, frühzeitig erkannt und dadurch Verletzungen der Harnröhre vermieden werden.

Eine günstige Ausführung einer erfindungsgemäßen Spritze eignet sich auch zur ausschließlichen Druckmessung bzw. -kontrolle beispielsweise einer Flüssigkeit. Hierfür wird der Spritzenzylinder wie auch der Kanülenansatz der Spritze zunächst entlüftet und sichergestellt, daß der Kolben während der Messung nicht im Spritzenzylinder zurückrutschen kann, z.B. durch eine schwergängige zirkuläre Dichtlippe. Durch den zu messenden Druck, unter dem die Flüssigkeit steht, wird diese in den Meßkanal gedrückt. Man kann nun am Flüssigkeitsstand im Meßkanal wieder einen relativen oder aufgrund einer Kalibrierung auch absoluten Druckwert ablesen.

Durch eine Weiterentwicklung kann eine erfindungsgemäße Spritze auch zur druckkontrollierten Injektion eines Gases und, im Vergleich zu konventionellen Manometern, einfaches, kompaktes, ausreichend genaues und billiges Gas-Druckmeßinstrument verwendet werden, z.B. um einen "Cuff" (Ballon, mit dem ein in der Luftröhre plazierter Endotrachealtubus gegen die Luftröhrenwand abgedichtet wird) mit einem bestimmten Druck aufzublasen und diesen Druck auch weiter zu überwachen. Hierzu wird in die vordere, erste Öffnung des Meßkanales einer Spritze ein zur Wand des Meßkanales luftdicht schließendes, in diesem aber dennoch leicht verschiebbares Trennungsmittel, z.B. ein elastischer Sotpfen, beispielsweise aus Gummi oder Tropfen Öl, eingebracht, das die zum Aufblasen des "Cuffs" in die Spritze aufgezogene Luft von der im Meßkanal und im Puffer des Kolbens befindlichen Luft trennt. Wird nun die im Spritzenzylinder befindliche Luft komprimiert, so wird das Trennungsmittel im Meßkanal nach hinten gedrückt und dadurch das im Meßkanal und Puffer des Kolbens befindliche Luftvolumen komprimiert. Aus der Position des Trennungsmittels zu einer auf dem Meßkanal angebrachten Skala - das Trennungsmittel fungiert somit auch als Zeiger - ist der Druck entweder als relativer oder, nach Eichung, auch absoluter Wert abzulesen. Durch Veränderung des Luftvolumens im Puffer des Kolbens kann auch bei dieser Spritze der Druckanzeigebereich feinfühlig eingestellt werden oder durch Verschluß der hinteren, zweiten Öffnung des Meßkanales mit einem geeigneten Verschlußstück erheblich verändert werden.

Nach dem Prinzip einer in den Kolben integrierten Dämpfungs- und Druckmeßeinrichtung sind weitere Anwendungen möglich: Unter Verwendung eines Zylinders ohne Ausflußöffnung sind auch Dämpfungselemente, z.B. kleines Luftvolumen im Puffer des Kolbens, relativ großes Luftvolumen im Meßkanal, oder auch Druckmeßinstrumente, z.B. großes Luftvolumen im Puffer, kleines Luftvolumen in einem langen und dünnen Meßkanal, einsetzbar.

Einsatzmöglichkeiten der Erfindung werden im folgenden aufgezeigt. Die Darstellung der Erfindung in medizinischen Bereichen schränkt die Anwendung eines erfindungsgemäßen Verfahrens und einer erfindungsgemäßen Vorrichtung in anderen Anwendungsbereichen nicht ein:
a. Spinalanästhesie: Eine druck- und somit geschwindigkeitskontrollierte Injektion von Lokalanästhetika reduziert die Gefahr von Nervenschäden durch den Injektionsstrahl.
   Eine gleichmäßige, insbesondere vergleichmäßigte Injektion von hyper- oder hypobaren Lokalanästhetika ermöglicht einseitige Spinalanästhesien.
   Eine gleichmäßige bzw. vergleichmäßigte Injektion von hyperbaren Lokalanästhetika ermöglicht hinsichtlich der Anästhesieausbreitung besser zu steuernde beidseitige Spinalanästhesien, z.B. Sattelblockanästhesien.
b. Langsame und gleichmäßige Injektion eines Fluides, z.B. einem Medikament, die aufgrund ihrer Auswirkungen auf den Kreislauf oder einer stark gefäßreizenden Wirkung nicht schnell injiziert werden dürfen: z.B. Propofol = intravenöses Narkotikum; eine mikroprozessorgesteuerte Pumpe für das korrekte, d.h. nicht zu schnelle Spritzen der Narkosedosis kann entfallen.
c. Druckkontrollierte Injektion von Ultraschallkontrastmitteln ("microbubble"-Lösungen): Bei zu großem Injektionsdruck werden die kleinen Gasblasen zerstört.
d. Einsatz als Dosiervorrichtung, insbesondere Mikrodosiervorrichtung, z.B. Injektion kleiner Mengen eines Fluides, beispielsweise im medizinischen Bereich eines stark kreislaufwirksamen Medikamentes, "Vorfüllen" der Infusionsschenkel von zentralvenösen Kathetern: In den entsprechend groß- bzw. kleinvolumig ausgelegten Meßkanal wird zunächst bei verschlossener Austrittsöffnung des Spritzenzylinders, z.B. durch einen Drei-Wege-Hahn, das zu injizierende Flüssigkeitsvolumen (hoch)gespritzt. Nach Öffnen der Austrittsöffnung des Spritzenzylinders wird dann exakt das im Meßkanal befindliche Volumen bei kleinem Injektionswiderstand schnell ("Bolus") oder bei großem Injektionswiderstand, z.B. durch ein Durchflußreduzierstück, langsam injiziert.
e. Die Spritze wird als "Druckfühler" benutzt, beipielsweise in der Periduralanästhesie: Gegen den vergleichsweise hohen intraligamentären Injektionswiderstand wird die Spritze (rückwärtig offener Meßkanal = großes Luftvolumen = low-pressure-Variante) durch Vorschieben des Kolbens unter Druck gesetzt. Mit Erreichen des Periduralraumes fällt die Flüssigkeitssäule im Meßkanal der Spritze aufgrund des plötzlich nachlassenden Injektionswiderstandes schlagartig.
   Diese Technik erlaubt im Vergleich zur klassischen Loss-of-resistance-Technik ein beidhändiges, kontrolliertes Vorschieben der Periduralnadel und liefert bei Erreichen des Periduralraumes ein eindeutiges optisches Signal.
   Aber auch eine konventionelle Loss-of-resistance-Spritze kann mit einer einfachen Druckmeßeinrichtung im Kolben ausgerüstet werden (rückwärtig verschlossener Meßkanal = kleines Luftvolumen = high-pressure-Variante).
   Die Vorteile der beiden Loss-of-resistance-Techniken - mit Luft bzw. Flüssigkeit oder einem anderen Fluid - lassen sich so vorteilhaft kombinieren: Einerseits das "Gefühl" und die "Beobachtung" des federnden Kolbens der "Luft"-Technik, solange sich die Spitze der Periduralnadel noch intraligamentär befindet und dem Verlust des Federns, wenn der Periduralraum erreicht ist. Andererseits die, aus der zunehmenden Drucksteigerung bei weiterem Einschieben des Kolbens resultierende, Kraftschlüssigkeit der "Flüssigkeits"-Technik und somit das "direkte Fühlen" des mit Erreichen des Periduralraumes plötzlich nachlassenden Injektionswiderstandes. Durch die Kombination der beiden Loss-of-resistance-Techniken werden Änderungen des Injektionswiderstandes nicht nur subjektiv ("Fühlen" des Widerstandsverlustes) sondern auch objektiv für den ausführenden Anästhesisten wie auch einen Beobachter beurteilbar (Druckmesssung).
   Zudem läßt sich vermeiden, daß unsterile Luft in den Periduralraum injiziert wird.
f. Druckkontrolliertes "Blocken", z.B. mit Flüssigkeit, von Ballonkathetern, z.B. Blasenkatheter, Embolektomiekatheter (Fogarty): Durch Messung des Druckes, mit dem der Ballon des Katheters "geblockt" wird, kann eine Fehllage bzw. kritische Lage des Ballons (an zu hohen Druckanstiegen) erkannt werden und die Gefahr eines Schadens, beispielsweise Verletzungen der Harnröhre oder eines Gefäßes, sinkt.
g. Druckkontrolliertes "Blocken", z.B. mit Luft, und kontinuierliche Druckkontrolle eines Tubuscuffs oder Pulmonaliskatheters (Swan-Ganz) mittels einer Spritze mit Trennungsmittel im Meßkanal.
h. Modifikation von Perfusor-Spritzen: Bei leerem Spritzenzylinder und somit Förderende der Perfusor-Pumpe stoppt die Injektion nicht schlagartig, sondern nimmt durch Förderung des im Meßkanal befindlichen Volumens langsam ab. Bei einem entsprechend lang ausgelegtem Meßkanal, z.B. aufgewickelter Schlauch, ist ein erhebliches "Puffervolumen" möglich.
   Perfusor-Spritzen, insbesondere Perfusor-Spritzen mit stark kreislaufwirksamen Medikamenten (z.B. Katecholamintherapie bei kritischen Intensivpatienten) können so selbst mit nur einer Perfusor-Pumpe gefahrlos gewechselt werden.
   Darüberhinaus ist auch eine Förderkontrolle möglich, da bei Verschluß, z.B. eine abgeknickte Infusionsleitung, die Flüssigkeit weiter in den Meßkanal gedrückt wird. Eine Drucksteigerung kann dadurch schneller, ggfs. durch einen Sensor (Spule, Fotozelle, Leitungskontakt etc.) als über den Verschlußdruckalarm der Perfüsor-Pumpe erkannt werden.
   Ferner können unbeabsichtigte Bolus-Gaben, beispielsweise durch zu hohen Förderdruck der Perfusor-Pumpe, vermieden werden, da der mit der gewünschten Fördergeschwindigkeit korrelierende Druck vor dem "Anschalten" der Perfusor-Spritze exakt eingestellt bzw. kontrolliert werden kann.
i. Druck- bzw. Förderkontrolle auch von konventionellen Perfusor-Spritzen: Hierbei wird eine erfindungsgemäße Spritze als "Druckmesser" mittels eines Drei-Wege-Hahnes parallel an die Perfusor-Spritze angeschlossen.
j. Druckkontrolle von Druck- oder auch Spülinfusionen, im medizinischen Bereich beispielsweise bei arterieller oder zentralvenöser Druckmessung: Mittels einer parallel zum Infusionsschenkel angeschlossenen erfindungsgemäßen Spritze (high-pressure-Variante, vgl. e.) wird der durch eine einfache Druckmanschette erzeugte und aufrechterhaltene Druck direkt im Infusionssystem gemessen und nicht indirekt über ein konventionelles, manchmal reparaturanfälliges Manometer, das den Druck in der Druckmanschette anzeigt.
k. Identifikation bzw. Auffinden von Höhlen, beispielsweise Körperhöhlen: Pleurapunktion zur Einlage einer Drainage oder eines Katheters, Punktion des Bauchraumes im Rahmen endoskopischer Eingriffe, Tracheapunktion im Rahmen einer Bougierungs-Tracheotomie, Punktion der Harnblase zur Einlage eines suprapubischen Blasenkatheters. Die Spritze wird als "Druckfühler" eingesetzt (vgl. e.). Die bei Nachlassen des Injektionswiderstandes plötzliche Injektion des im Meßkanal der Spritze befindlichen Flüssigkeitsvolumens führt zu einem Injektionsstrahl, der verletzliche Strukturen, z.B. Lunge oder Darm, von der Nadelspitze wegdrückt und somit die Gefahr der Verletzung dieser Strukturen senkt.
l. Messung eines Druckes oder einer Druckschwankung, im medizinischen Bereich beispielsweise des zentralvenösen oder auch arteriellen (Mittel-)Druckes: Die Spritze ist verglichen mit der sonst verwendeten Steigleitung zur Messung des zentralvenösen (MittelDruckes in Zentimeter Wassersäule eine handlichere und kompaktere Druckmeßeinrichtung.
   Der Meßkanal kann durch wechselnde Querschnitte in einen vorderen, genauen Niederdruckmeßbereich (Messung des zentralvenösen (Mittel-)Druckes = langes und kleinlumiges Meßkanalstück) und einen hinteren, mit anderen Meßtoleranzen arbeitenden Hochdruckmeßbereich (Messung des arteriellen (Mittel)Druckes = kurzes und großlumiges Meßkanalstück) unterteilt werden.
   Es ist auch eine Messung von negativen Drücken, also Unterdrücken, möglich: Nach dem Aufziehen der Flüssigkeit in den Spritzenzylinder wird der Kolben gegen den atmosphärischen Druck geöffnet, z.B. durch einen Schieber oder zwei gegeneinander verdrehbare Öffnungen im Kolben und Verschlußstück. Nachdem bei verschlossener Austrittsöffnung des Spritzenzylinders durch Vorschieben des Kolbens etwas Flüssigkeit in den Meßkanal gedrückt worden ist, beispielsweise bis zu einer Nullmarkierung, z.B. der Hälfte des Meßkanales, wird der Kolben wieder gegen den atmosphärischen Druck luftdicht verschlossen. Jetzt können sowohl positive wie auch negative Drücke gemessen werden.
   Die Spritze ist im Gegensatz zur Schlauchleitung, die immer gegen den atmosphärischen Druck offen ist, ein geschlossenes System; dies hat hygienische Vorteile.
m. Punktionen von Flüssigkeitsräumen, im medizinischen Bereich z.B. Blutgefäße, Harnblase zur Einlage eines suprapubischen Blasenkatheters, Punktion eines Pleuraergusses, Aszitespunktion: Eine für die Messung negativer Drücke vorbereitete Spritze (s. l.) wird luftdicht an die Punktionskanüle angesetzt. Nach Einstechen der Punktionskanüle wird der Kolben soweit im Spritzenzylinder zurückgezogen, bis der gewünschte Unterdruck eingestellt ist. Bei entsprechender Auslegung der zirkulären Dichtlippe des Kolbens wird der Kolben dabei in seiner Position im Spritzenzylinder und somit der eingestellte Unterdruck gehalten. Erreicht die Spitze der Punktionskanüle den Flüssigkeitsraum, steigt der Flüssigkeitsspiegel im Meßkanal schlagartig an, maximal bis zum Druckausgleich.
   Ein solches druckkontrolliertes Aspirieren (= Ansaugen) kann verhindern, daß, wie durch starke Aspiration sonst möglich, bei Punktion von elastischen oder nachgebenden Wänden andere elastische Wände angesaugt werden, wie im medizinischen Bereich beispielsweise bei Punktion eines Blutgefäßes die Gefäßwand an die Kanülenöffnung gesaugt werden kann. Da in einem solchen Fall kein Blut zu aspirieren ist, nimmt man fälschlicherweise an, daß man das Blutgefäß noch nicht erreicht oder schon wieder verlassen hat. Dadurch steigt die Zahl von Fehl- bzw. Mehrfachpunktionen.
   Zusätzlich ist eine Druckmessung im punktierten Gefäß möglich. So lassen sich, bei dem Versuch eine zentrale Vene zu punktieren, unbeabsichtigte Punktionen einer Arterie unmittelbar erkennen. Nach der Punktion kann durch Injektion von Flüssigkeit, z.B. Kochsalzlösung, die Punktionskanüle unmittelbar gespült werden; ein Verstopfen der Kanüle durch gerinnendes Blut wird so verhindert.

Weitere Vorteile und Eigenschaften der Erfindung werden anhand der in den Zeichnungen dargestellten bevorzugten Ausführungsformen erläutert.

Es zeigen:
- Fig. 1: eine Spritze mit in deren Kolben integriertem Puffer und Meßkanal zur vergleichmäßigten und druckkontrollierten Injektion eines Fluides, z.B. einer Flüssigkeit oder eines Gases, im Halbschnitt,
- Fig. 2: eine Spritze nach Fig. 1 mit einer aufgesteckten großlumigen Kanüle im Halbschnitt, während ein Fluid durch die großlumige Kanüle in den Spritzenzylinder aufgezogen wird,
- Fig. 3: eine Spritze nach Fig. 1 mit einer aufgesteckten kleinlumigen Kanüle im Halbschnitt, während ein im Spritzenzylinder befindliches Fluid durch die kleinlumige Kanüle injiziert wird,
- Fig. 4: eine Spritze nach Fig. 3 mit einem angesetzten Durchflußreduzierstück und einer aufgesteckten großlumigen Kanüle (gestrichelt dargestellt) im Halbschnitt, während ein im Spritzenzylinder befindliches Fluid durch das Durchflußreduzierstück injiziert wird,
- Fig. 5: eine Spritze nach Fig. 3 im Halbschnitt mit einem im Kolben verschiebbaren, die hintere Öffnung des Meßkanales nicht verschließenden Verschlußstück,
- Fig. 6: eine Spritze nach Fig. 5 im Halbschnitt mit einem in den Kolben eingeschobenen, die hintere, zweite Öffnung des Meßkanales verschließenden Verschlußstück,
- Fig. 7: eine Spritze nach Fig. 1 im Halbschnitt mit einem die vordere, erste Öffnung des Meßkanales dicht verschließenden, im Meßkanal leicht verschiebbaren Trennungsmittel,
- Fig. 8: eine Spritze nach Fig. 3 mit angesetzter Schlauchleitung im Halbschnitt, während ein im Spritzenzylinder befindliches Fluid, z.B. Luft, in eine Schlauchleitung injiziert wird,
- Fig. 9: ein Diagramm mit der Darstellung der Flußrate über dem Druck in Abhängigkeit von als Durchflußwiderstand verwendeten dünnen Spinalnadeln und
- Fig. 10: ein Diagramm mit der Darstellung des resultierenden Druckes über der Kompression eines kompressiblen Mediums in verschieden großen Puffern eines Kolbens einer erfindungsgemäßen Vorrichtung.

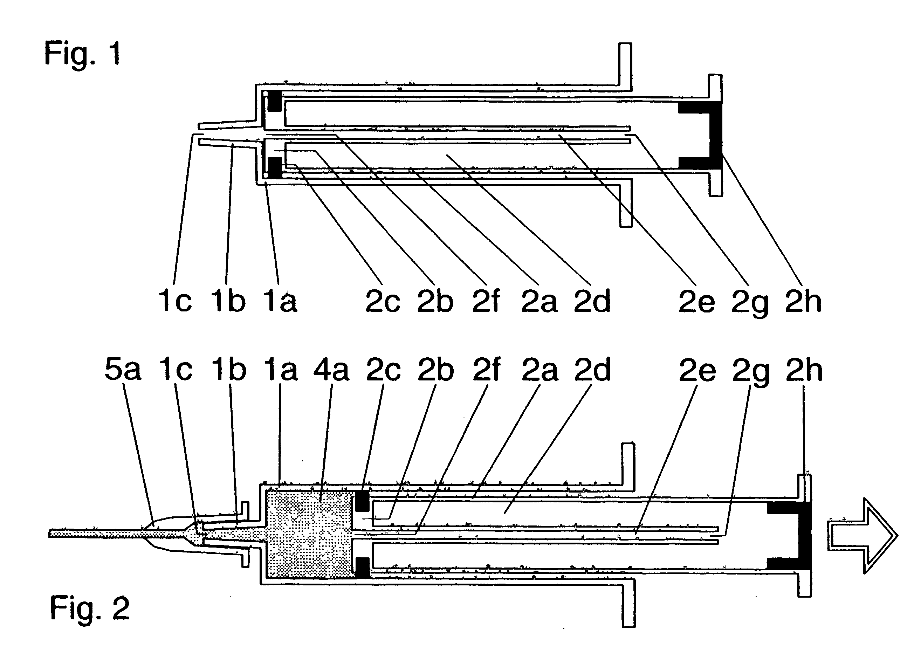

Fig. 1 zeigt eine vorteilhafte Ausführungsform der Erfindung in der Form einer Spritze mit einem, im Spritzenzylinder 1a liegenden, innen hohlen Kolben 2a, der, an der in den Spritzenzylinder 1a eingeschobenen Seite, durch eine im Zylinderstück 2b des Kolbens 2a liegende zirkuläre Dichtlippe 2c zur Innenwand des Spritzenzylinders 1a abgedichtet und an der aus dem Spritzenzylinder 1a ragenden Seite mit einem Verschlußstück 2h luftdicht verschlossen ist. Der Spritzenzylinder 1a und der Boden des Kolbens 2a bilden zusammen einen Behälter von variablem, nämlich von der Stellung des Kolbens 2a abhängendem Volumen. Von der Mitte des Zylinderstückes 2b führt, über eine vordere, erste 2f und hintere, zweite Öffnung 2g, ein zu beiden Seiten hin offener Meßkanal 2e in den Puffer 2d des Kolbens 2a. Der Puffer 2d und der Meßkanal 2e sind mit einem kompressiblen Medium, in diesem Fall Luft, gefüllt.

Fig. 2 zeigt eine am Kanülenansatz 1b des Spritzenzylinders 1a aufgesteckte, in der Länge gekürzt dargestellte, großlumige Kanüle 5a, die einen mit dem Austritt 1c des Spritzenzylinders 1a vergleichbaren Innendurchmesser und somit relativ niedrigen Durchflußwiderstand hat. Dieses ist für das Aufziehen einer Flüssigkeit 4a, z.B. eines Medikamentes, hilfreich. Durch Zurückziehen des Kolbens, durch den Pfeil angedeutet, wird die Flüssigkeit 4a in den Spritzenzylinder 1a aufgezogen, dabei verlegt die Flüssigkeit 4a die mittig im Zylinderstück 2b des Kolbens liegende vordere, erste Öffnung 2f des Meßkanales 2e und schließt das im Meßkanal 2e und Puffer 2d des Kolbens 2a befindliche kompressible Medium nach außen ab.

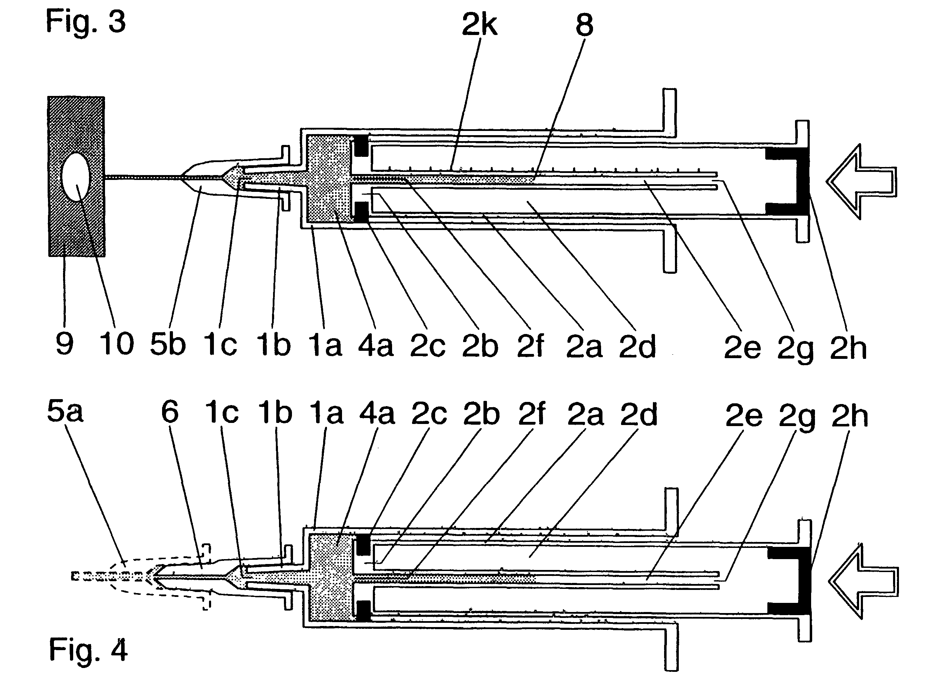

Fig. 3 zeigt eine am Kanülenansatz 1b des Spritzenzylinders 1a aufgesteckte, in der Länge gekürzt dargestellte, kleinlumige Kanüle 5b, die einen kleinen Innendurchmesser und somit relativ hohen Durchflußwiderstand hat. Aufgrund eines deutlich niedrigeren Durchflußwiderstandes der vorderen, ersten Öffnung 2f des Meßkanales 2e wird nun durch Einschieben des Kolbens 2a, durch den Pfeil angedeutet, bei Überschreiten eines bestimmten, auf der Flüssigkeit 4a lastenden Druckes Flüssigkeit 4a in den Meßkanal 2e gedrückt, das kompressible Medium aus dem Meßkanal 2e in den Puffer 2d des Kolbens 2a verdrängt und dadurch das kompressible Medium komprimiert. Nimmt nun der durch das Einschieben des Kolbens 2a auf der Flüssigkeit 4a lastende Druck ab, so wird die Flüssigkeit 4a durch das komprimierte kompressible Medium unter Dekompression wieder aus dem Meßkanal 2e gedrückt. Die im Zylinderstück 2b liegende zirkuläre Dichtlippe 2c ist so dimensioniert, daß ein ruckfreies Einschieben des Kolbens 2a in den Spritzenzylinder 1a möglich ist und der Kolben 2a auch bei vollständiger Entlastung des auf den Kolben 2a drückenden Fingers seine Einschiebtiefe im Spritzenzylinder 1a behält. So werden einerseits Drucksteigerungen durch ein zu schnelles Einschieben des Kolbens 2a in den Spritzenzylinder 1a gedämpft, andererseits wird der Fluß der Flüssigkeit 4a durch eine kleinlumige Kanüle 5b vergleichmäßigt. Fig. 3 veranschaulicht auch den Einsatz der Spritze zum Auffinden eines Hohlraumes 10 in einem Körper 9. Dazu wird die Kanüle 5b langsam in den Körper 9 eingeführt, während gleichzeitig das Fluid 4a unter einem bestimmten Druckk gehalten wird. Erreicht die Spitze der Kanüle 5b den Hohlraum 10, so kann das Fluid 4a plötzlich leichter aus der Kanüle 5b austreten, was einen Druckabfall im Spitzenzylinder 1a zur Folge hat, der durch ein plötzliches Absinken der Grenzschicht 8 im Meßkanal 2e leicht erkennbar ist. Bei Verwendung einer großlumigen Kanüle 5b ist der Druckabfall bei ansonsten gleichen Bedingungen gegenüber einer kleinlumigen Kanüle 5b deutlicher zu bemerken.

Fig. 4 zeigt eine Fig. 3 entsprechende Situation, wobei an den Kanülenansatz 1b des Spritzenzylinders 1a ein Durchflußreduzierstück 6 angesetzt ist, das den für eine langsame und vergleichmäßigte Injektion der Flüssigkeit 4a wichtigen, relativ hohen Durchflußwiderstand schafft. Bei Verwendung eines Durchflußreduzierstückes 6 kann so auch durch eine großlumige, gestrichelt angedeutete Kanüle 5a trotz ihres kleinen Durchflußwiderstandes langsam und vergleichmäßigt injiziert werden.

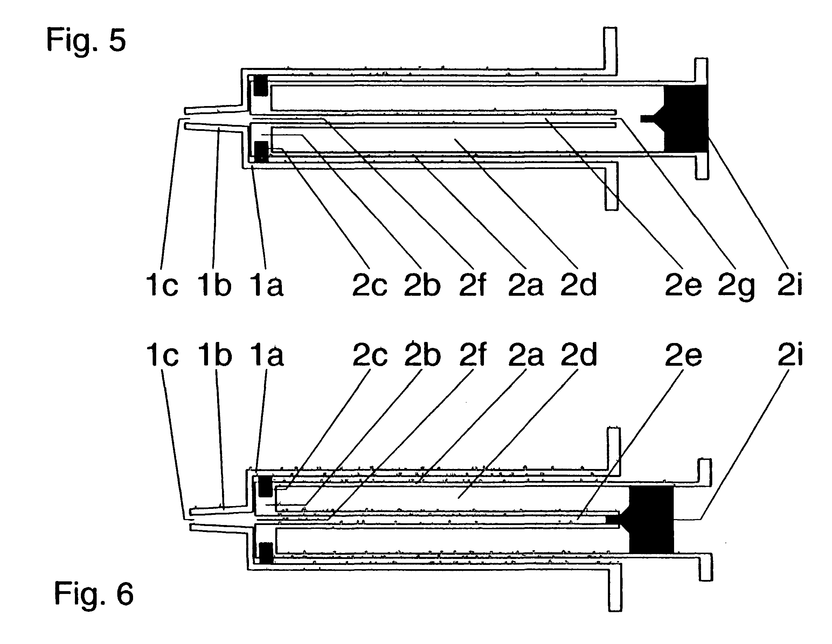

Fig. 5 zeigt eine Fig. 3 entsprechende Situation, wobei durch ein größeres Verschlußstück 2i das Volumen des kompressiblen Mediums im Meßkanal 2e und Puffer 2d des Kolbens 2a verkleinert worden ist. Durch Veränderung des im Meßkanal 2e und Puffer 2d befindlichen Volumens des kompressiblen Mediums lassen sich die Dämpfungseigenschaften der Spritze verändern. Hierdurch kann die Spritze an verschieden große Durchflußwiderstände unterschiedlicher Kanülen angepaßt werden, so daß ein bestimmter Flüssigkeitsstand im Meßkanal 2e immer einer bestimmten Injektionsgeschwindigkeit entspricht.

Fig. 6 zeigt eine Fig. 5 entsprechende Situation, wobei durch Einschieben des Verschlußstückes 2i in den Kolben 2a die hintere, zweite Öffnung 2g des Meßkanales 2e verschlossen worden ist. Durch diese erhebliche Verkleinerung auf das im Meßkanal 2e befindliche Volumen des kompressiblen Mediums wird auch das Dämpfungsverhalten der Sprite erheblich verändert. Hierdurch kann man mit der Spritze in zwei verschiedenen Dämpfungs- und Druckmeßbereichen arbeiten.

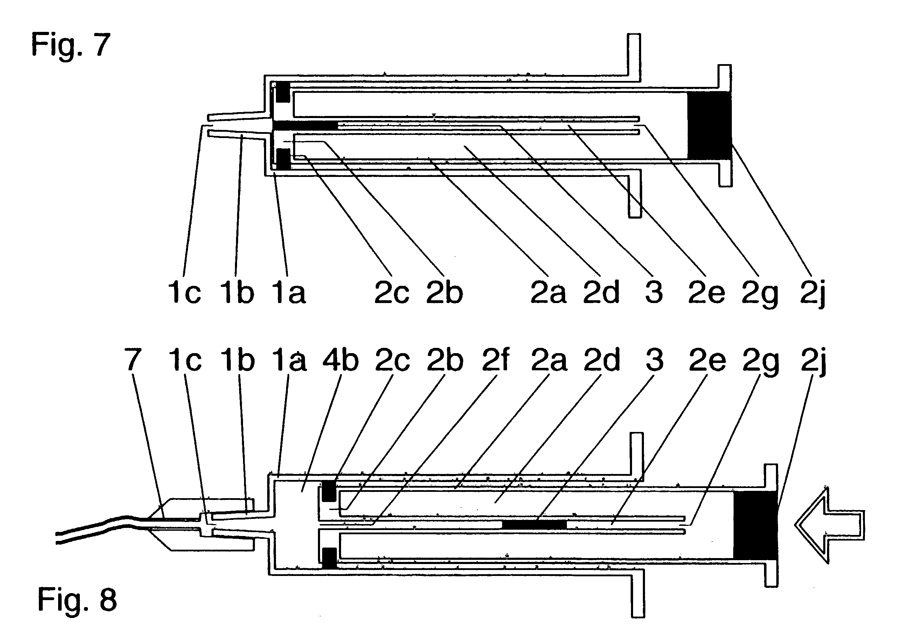

Fig. 7 zeigt eine Fig. 1 vergleichbare Situation, wobei durch ein Trennungsmittel 3, z.B. ein elastische Stopfen oder ein Tropfen Öl, das die vordere, erste Öffnung 2f des Meßkanales 2e verschließt, insbesondere luftdicht, aber dennoch im Meßkanal 2e leicht verschiebbar ist, das im Meßkanal 2e und Puffer 2d befindliche kompressible Medium nach außen abgeschlossen ist. Mit einer in dieser Weise modifizierten Spritze kann auch ein Gas 4b, z.B.Luft, druckkontrolliert injiziert werden.

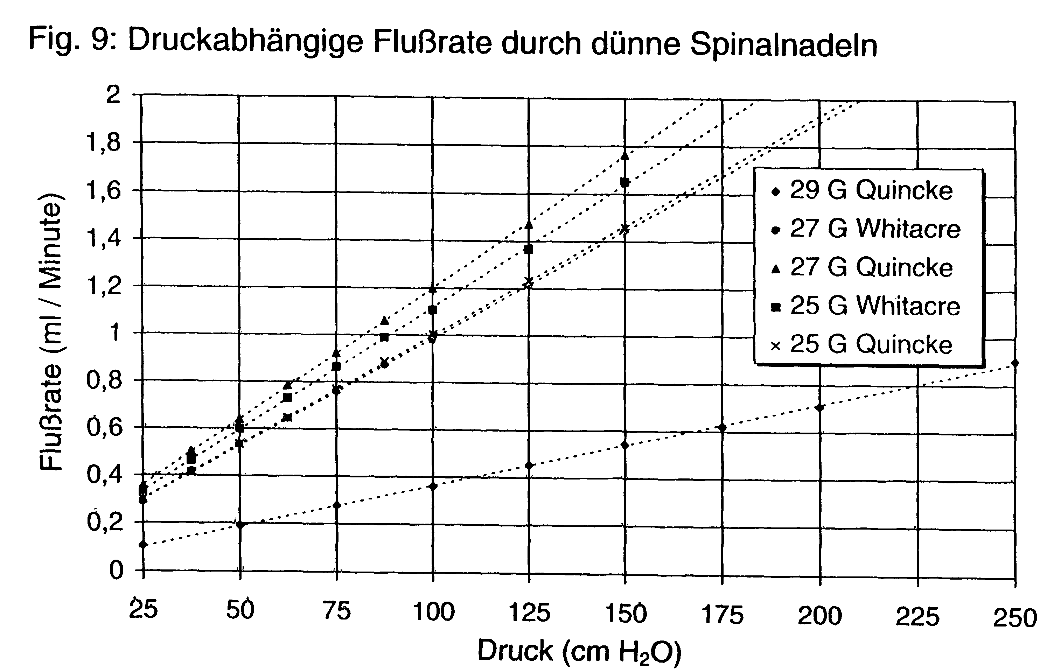

Fig. 8 zeigt eine Fig. 3 vergleichbare Situation, wobei an Stelle einer Flüssigkeit 4a, die mit einer solchen Spritze ebenfalls injiziert werden kann, ein Gas 4b, z.B. Luft, in eine Schlauchleitung 7 injiziert wird. Die Position des Trennungsmittels 3, z.B. ein elastischer Stopfen, ein Tropfen Öl, etc., im Meßkanal 2e zeigt den auf dem Gas 4b lastenden Druck während der Injektion, durch den Pfeil angedeutet, an. Die Druckmessung kann als Relativdruckmessung oder bei Eichung der Vorrichtung auch als Absolutwertmessung erfolgen. Das Verschlußstück 2j ist so dimensioniert, daß das Trennungsmittel 3 nicht oder nur schwer aus dem Meßkanal 2e herausgedrückt werden kann.

Fig. 9 zeigt ein Diagramm mit der Darstellung der Flußrate über dem Druck in Abhängigkeit von als Durchflußwiderstand verwendeten dünnen Spinalnadeln. Es zeigt, daß eine gleichmäßige, niedrige Flußrate nur schwer zu erzielen ist, da schon bei geringen Druckänderungen die Flußrate erheblich schwankt. Insbesondere bei Verwendung konventioneller Spritzen treten bei Injektion mit freier Hand solche Druckänderungen unkontrollierbar und ungedämpft auf.

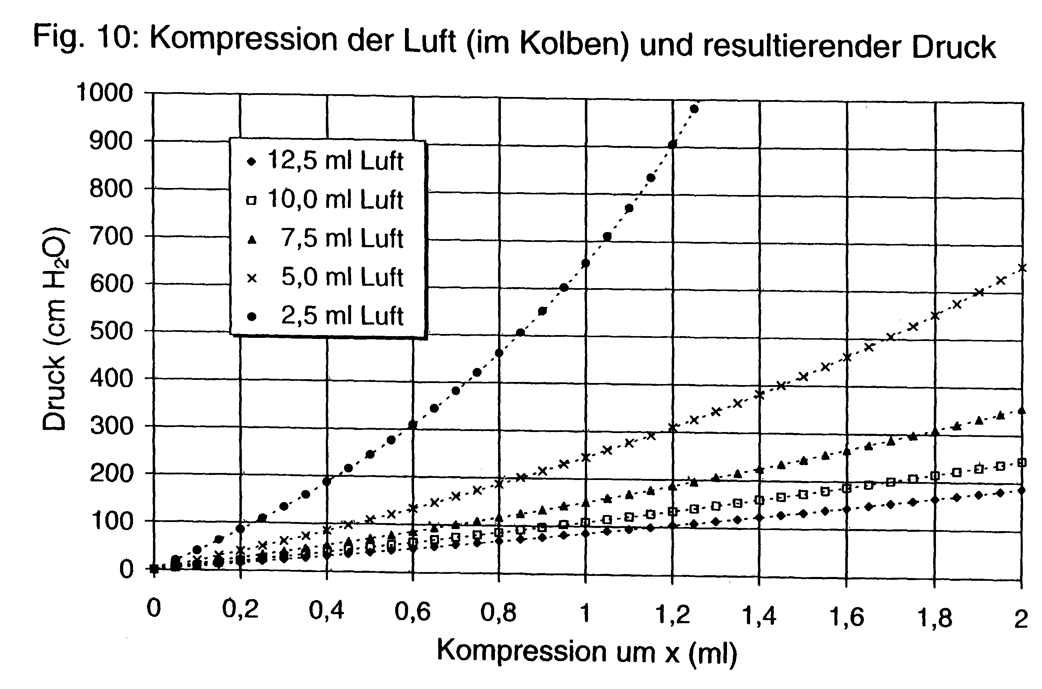

Fig. 10 zeigt ein Diagramm mit der Darstellung des resultierenden Druckes über der Kompression eines kompressiblen Mediums, in diesem Falle Luft, in einem Puffer eines Kolbens einer erfindungsgemäßen Vorrichtung. Es verdeutlicht die Dämpfungseigenschaften verschiedener Volumina des kompressiblen Mediums in einer erfindungsgemäßen Vorrichtung. So kann je nach gewünschter Dämpfung und der daraus resultierenden Vergleichmäßigung der Flußrate ein entsprechendes Puffervolumen in der Vorrichtung, beispielsweise über die Veränderung des Puffers im Kolben der Spritze, eingestellt werden.

Die vorliegende Erfindung erlaubt eine einfache visuelle oder meßtechnische Kontrolle des Druckes bei der Handhabung eines Fluides, wodurch insbesondere medizinische Maßnahmen, wie das Injizieren eines Fluides, wesentlich erleichtert und verbessert werden können.

### BEZUGSZEICHENLISTE

- 1a: Behälter, Spritzenzylinder
- 1b: Kanülenansatz
- 1c: Austritt. Austrittsöffnung
- 2a: Druckerzeugungseinrichtung, Kolben
- 2b: Zylinderstück des Kolbens
- 2c: Dichtmittel, zirkuläre Dichtlippe, Reibungsmittel
- 2d: Puffer
- 2e: Meßkanal
- 2f: vordere, erste Öffnung
- 2g: hintere, zweite Öffnung
- 2h, 2i, 2j: verschiedene Verschlußstücke
- 2k: Meßeinrichtung, Skala
- 3: Trennungsmittel (elastischer Stopfen, Tropfen Öl)
- 4a, 4b: Fluid (Flüssigkeit, Gas)
- 5a, 5b: verschiedene Kanülen
- 6: Durchflußreduzierstück
- 7: Schlauchleitung
- 8: Grenzschicht zwischen Fluid und kompressiblem Medium
- 9: Körper
- 10: Hohlraum

## Patentansprüche

1. Verfahren zur druckkontrollierbaren Handhabung eines Fluides (4a; 4b), ausgenommen Verfahren zur chirurgischen oder therapeutischen Behandlung des menschlichen oder tierischen Körpers und Diagnostizierverfahren, die am menschlichen oder tierischen Körper vorgenommen werden, wobei
- das in einem Behälter (1a) von variablem Volumen befindliche Fluid (4a; 4b) durch einen auf das Fluid (4a; 4b) einwirkenden Druck an einem Austritt(1c) mit diesem Druck zur Verfügung gestellt wird,
- der Behälter (1a) mit einem Kanal von vorgegebenem Querschnitt in Verbindung steht,
- der Kanal mit einem Puffer (2d) kommuniziert, wobei der Puffer (2d) ein Volumen hat,
- der Kanal und der Puffer (2d) mit einem kompressiblen Medium gefüllt sind, **dadurch gekennzeichnet,**
**daß** das Fluid (4a; 4b) entsprechend dem einwirkenden Druck, dem Querschnitt des einen Meßkanal (2e) bildenden Kanals und der vorgegebenen Größe des Volumens des Puffers (2d) zumindest teilweise in den Meßkanal (2e) eindringt, wobei während der Handhabung des Fluids (4a; 4b) die Position der Grenzschicht (8) zwischen dem Fluid (4a; 4b) und dem kompressiblen Medium in dem Meßkanal (2e) beobachtet oder gemessen wird und das Handhaben das Erzeugen eines gewissen Druckes in dem Behälter (1a) und das Bewegen einer mit dem Auslaß (1c) verbundenen Kanüle (5b) in einen Körper (9) umfaßt, um ein Gebiet (10) in dem Körper (9) aufzufinden und zu identifizieren, welches einen von dem erzeugten gewissen Druck abweichenden Druck aufweist, indem die Position der Grenzschicht (8) beobachtet wird.

2. Verfahren nach Anspruch 1, wobei das Handhaben ein druckkontrollierbares Injizieren eines Fluides (4a; 4b) durch den Austritt (1c) aus dem Behälter (1a) in einen Körper (9) ist.

3. Verfahren nach Anspruch 1 oder 2, wobei der auf das Fluid (4a; 4b) einwirkende Druck durch einen in den Behälter (1a) schiebbaren Kolben (2a) erzeugt wird, der dichtend (2c) an den Innenwänden des Behälters (1a) geführt ist.

4. Verfahren nach Anspruch 1, wobei der auf das Fluid (4a; 4b) einwirkende Druck in Abhängigkeit von der Position der Grenzschicht (8) eingestellt und/oder geregelt wird.

5. Vorrichtung zum druckkontrollierbaren Handhaben eines Fluides (4a; 4b), mit
- einem Behälter (1a) von variablem Volumen zur Aufnahme des Fluides (4a; 4b),
- einer Druckerzeugungseinrichtung (2a) zur Erzeugung eines auf das Fluid (4a; 4b) einwirkenden Druckes,
- einem Austritt (1c) zum Einlaß und/oder Auslaß des Fluides (4a; 4b),
- einem Kanal von vorgegebenem Querschnitt, der mit dem Behälter (1a) in Verbindung steht, **dadurch gekennzeichnet, daß** die Vorrichtung einen Puffer (2d) von vorgegebenem Volumen hat, der mit dem Kanal als Meßkanal (2e) kommuniziert und der zum Zeitpunkt des druckkontrollierten Handhabens bis auf eine mögliche Höhe eines Fluides im Querschnitt des Meßkanals durch feste Wände beschränkt wird,
wobei der Meßkanal (2e) und der Puffer (2d) mit einem kompressiblen Medium gefüllt sind. kompressiblen Medium gefüllt sind.

6. Vorrichtung nach Anspruch 5, wobei die Druckerzeugungseinrichtung ein in den Behälter (1a) einschiebbarer Kolben (2a) ist, der zur Innenwand des Behälters (1a) durch Dichtmittel (2c) abgedichtet ist.

7. Vorrichtung nach Anspruch 6, wobei der Meßkanal (2e) in dem Kolben (2a) angeordnet ist und mit einer ersten Öffnung (2f) in dem vom Behälter (1a) und dem Kolben (2a) gebildeten Raum für das Fluid (4a; 4b) mündet.

8. Vorrichtung nach Anspruch 6 oder 7, wobei der Meßkanal (2e) mit einer zweiten Öffnung (2g) in einem Puffer (2d) mündet.

9. Vorrichtung nach Anspruch 6, 7 oder 8, wobei der Puffer (2d) innerhalb des Kolbens (2a) angeordnet ist.

10. Vorrichtung nach einem der Ansprüche 6 bis 9, wobei der Kolben (2a) und/oder der Meßkanal (2e) eine Meßeinrichtung (2k) zur Messung des Druckes des Fluides (4a; 4b) aufweist.

11. Vorrichtung nach einem der Ansprüche 7 bis 10, wobei die Wände des Behälters (1a), des Kolbens (2a) und des Meßkanals (2e) durchsichtig sind und eine dieser Wände, vorzugsweise die des Meßkanals (2e), eine Skala (2k) zur Positionsbestimmung der Grenzschicht (8) zwischen Fluid (4a; 4b) und kompressiblem Medium in dem Meßkanal (2e) aufweist

12. Vorrichtung nach einem der Ansprüche 6 bis 11, wobei das kompressible Medium und das im Behälter (1a) befindliche Fluid (4a; 4b) durch ein Trennungsmittel (3), das sich nicht mit dem Fluid (4a; 4b) und dem kompressiblen Medium mischt, voneinander getrennt sind, z. B. durch einen elastischen Stopfen oder einen Tropfen Öl.

13. Vorrichtung nach Anspruch 12, wobei das Trennungsmittel (3) in dem Meßkanal (2e) dichtend, aber mit im Vergleich zu den übrigen auftretenden Kräften vernachlässigbarem Widerstand verschiebbar ist.

14. Vorrichtung nach einem der Ansprüche 6 bis 13, wobei der Puffer (2d) im Inneren des Kolbens (2a) und der Meßkanal (2e) zentrisch im Inneren des Puffers (2d) angeordnet sind.

15. Vorrichtung nach einem der Ansprüche 6 bis 13, wobei der Meßkanal (2e) exzentrisch im Kolben (2a) liegt.

16. Vorrichtung nach Anspruch 15, wobei der Meßkanal (2e) nahe einer Außenwand des Kolbens (2a) oder in der Außenwand des Kolbens (2a) liegt.

17. Vorrichtung nach einem der Ansprüche 6 bis 16, wobei die Größe des Volumens des Puffers (2d) veränderbar ist.

18. Vorrichtung nach Anspruch 17, wobei das im Puffer (2d) des Kolbens (2a) befindliche Volumen des kompressiblen Mediums durch ein im Kolben (2a) angeordnetes Verschlußstück (2h; 2i; 2j) festlegbar ist.

19. Vorrichtung nach Anspruch 18, wobei das Verschlußstück (2b; 2i; 2j) beweglich und das Volumen des Puffers (2d) dadurch einstellbar ist.

20. Vorrichtung nach einem der Ansprüche 14 bis 16, wobei das im Meßkanal (2e) befindliche kompressible Medium durch ein Verschlußstück (2i) vom kompressiblen Medium, das sich im Puffer (2d) des Kolbens (2a) befindet, trennbar ist.

21. Vorrichtung nach einem der Ansprüche 5 bis 20, wobei der Puffer (2d) eine verschließbare Verbindungsöffnung zur Umgebungsatmosphäre aufweist.

22. Vorrichtung nach einem der Ansprüche 5 bis 21, wobei das kompressible Medium ein Gas ist, insbesondere Luft.

23. Vorrichtung nach einem der Ansprüche 5 bis 22, wobei die Vorrichtung im wesentlichen zylindrisch aufgebaut ist.

24. Vorrichtung nach einem der Ansprüche 5 bis 23, wobei die Vorrichtung eine Spritze, insbesondere für medizinische Anwendungen, ist.

25. Vorrichtung nach einem der Ansprüche 5 bis 24, wobei Reibungsmittel (2c) vorhanden sind, die den Kolben (2a) in jeder durch Kraft von außen eingestellten Stellung relativ zum Behälter (1a) auch bei Entlastung von dieser Kraft festhalten.

26. Vorrichtung nach einem der Ansprüche 5 bis 25, wobei das Volumen des Puffers (2d) mindestens 10, vorzugsweise 100, mal so groß ist wie das Volumen des Meßkanals (2c).

27. Vorrichtung nach einem der Ansprüche 5 bis 26, wobei der Austritt (1c) oder eine auf einen Kanülenansatz (1b) aufgesetzte Kanüle (5b) oder ein Durchflußreduzierstück (6) einen kleineren Austrittsquerschnitt aufweist als der Querschnitt des Meßkanals (2e).

28. Vorrichtung nach einem der Ansprüche 5 bis 27, wobei der Querschnitt des Austritts (1c), der Querschnitt des Meßkanals (2e) und das Volumen des Puffers (2d) je nach Anwendungsfall und den dabei benötigten Drücken so dimensioniert sind, daß das Fluid (4a; 4b) bei der Handhabung zumindest teilweise in den Meßkanal (2e) eindringt.

## Claims

1. A method for pressure-controllable handling of a fluid (4a; 4b) except methods for treating surgically or therapeutically the human or animal body and diagnostic procedures which are undertaken on the human or animal body, wherein
- the fluid (4a; 4b), located in a container (1a) of variable volume is made available by pressure at an outlet (1c) by means of said pressure acting upon the fluid (4a; 4b),
- the container (1a) is connected to a channel with a predetermined cross-section,
- the channel communicates with a buffer (2d), the buffer (2d) having a volume,
- the channel and the buffer (2d) are filled with a compressible medium, **characterized in that**
there is at least partial ingress of the fluid (4a; 4b) into the measuring channel (2e), dependent upon the action of the pressure, the cross-section of the channel defining a measuring channel (2e) and the predetermined size of the volume of the buffer (2d), wherein during handling of the fluid (4a; 4b) the position of the boundary layer (8) between the fluid (4a; 4b) and the compressible medium in the measuring channel (2e) is observed or measured and wherein handling includes the production of a certain pressure in the container (1a) and the movement of a cannula (5b) connected to the outlet (1c) into a body (9) in order to locate and to identify an area (10) in the body (9) which has a pressure different from the certain pressure produced, in which the position of the boundary layer (8) is observed.

2. Method according to claim 1, wherein the handling is pressure-controllable injection of a fluid (4a; 4b) through the outlet (1c) from the container (1a) into a body (9).

3. Method according to claim 1 or 2, wherein the pressure acting upon the fluid (4a; 4b) is produced by a plunger (2a) which can be pushed into the container (1a), which is guided in a sealed manner (2c) along the interior walls of the container (1a).

4. Method according to claim 1, wherein the pressure acting upon the fluid (4a; 4b) is adjusted and/or regulated dependent upon the position of the boundary layer (8).

5. Device for pressure-controllable handling of a fluid (4a; 4b) with
- a container (1a) of variable volume for receiving the fluid (4a; 4b),
- a pressure producing means (2a) for producing a pressure acting upon the fluid (4a; 4b),
- an outlet (1c) for the entrance and/or exit of the fluid (4a; 4b);
- a channel with a predetermined cross-section, which is connected to the container (1a),
**characterized in that** the device has a buffer (2d) with a predetermined volume, which communicates with the channel as measuring channel (2e) and which at the time of pressure-controlled handling is limited by fixed walls up to a possible height of a fluid in the cross-section of the measuring channel,
wherein the measuring channel (2e) and the buffer (2d) are filled with a compressible medium.

6. Device according to claim 5, wherein the pressure producing means is a plunger (2a) which can be pushed inside the container (1a), which is sealed with respect to the internal wall of the container (1a) by sealing means (2c).

7. Device according to claim 6, wherein the measuring channel (2e) is arranged in the plunger (2a) and leads with a first aperture (2f) into the space for the fluid (4a; 4b) formed by the container (1a) and the plunger (2a).

8. Device according to claim 6 or 7, wherein the measuring channel (2e) leads with a second aperture (2g) in a buffer (2d).

9. Device according to claim 6, 7 or 8, wherein the buffer (2d) is arranged inside the plunger (2a).

10. Device according to one of claims 6 to 9, wherein the plunger and/or the measuring channel (2e) is provided with a measuring means (2k) for measuring the pressure of the fluid (4a; 4b).

11. Device according to one of claims 6 to 10, wherein the walls of the container (1a), of the plunger (2a) and of the measuring channel (2e) are transparent and one of these walls, preferably that of the measuring channel (2e), is provided with a scale (2k) for determining the position of the boundary layer (8) between the fluid (4a; 4b) and the compressible medium in the measuring channel (2e).

12. Device according to one of claims 6 to 11, wherein the compressible medium and the fluid (4a; 4b) located in the container (1a) are separated from one another by a separating means (3) which does not mix with the fluid (4a; 4b) and the compressible medium, for example by means of an elastic stopper or a drop of oil.

13. Device according to claim 12, wherein the separating means (3) can be pushed into the measuring channel (2e) in a sealing manner, but with negligible resistance compared to the forces normally occurring.

14. Device according to one of claims 6 to 13, wherein the buffer (2d) is arranged in the interior of the plunger (2a) and the measuring channel (2e) in the interior of the buffer (2d).

15. Device according to one of claims 6 to 13, wherein the measuring channel (2e) lies eccentrically in the plunger (2a).

16. Device according to claim 15, wherein the measuring channel (2e) lies close to an outer wall of the plunger (2a) or in the outer wall of the plunger (2a).

17. Device according to one of claims 6 to 16, wherein the size of the volume of the buffer (2d) can be altered.

18. Device according to claim 17, wherein the volume of the compressible medium located in the buffer (2d) of the plunger (2a) can be determined by a sealing piece (2h; 2i; 2j) arranged in the plunger (2a).

19. Device according to claim 18, wherein the sealing piece (2h; 2i; 2j) is movable and in this way the volume of the buffer (2d) can be adjusted.

20. Device according to one of claims 14 to 16, wherein the compressible medium located in the measuring channel (2e) can be separated by means of a sealing piece (2i) from the compressible medium which is located in the buffer (2d) of the plunger (2a).

21. Device according to one of claims 5 to 20, wherein the buffer (2d) is provided with a sealable connecting aperture to the surrounding atmosphere.

22. Device according to one of claims 5 to 21, wherein the compressible medium is a gas, in particular air.

23. Device according to one of claims 5 to 22, wherein the device is constructed substantially in a cylindrical manner.

24. Device according to one of claims 5 to 23, wherein the device is a syringe, particularly for medical applications.

25. Device according to one of claims 5 to 24, wherein friction means (2c) are present which hold the plunger (2a) in any position relative to the container (1a) set by force applied from the outside, even when this force is removed.

26. Device according to one of claims 5 to 25, wherein the volume of the buffer (2d) is at least 10, preferably 100 times as large as the volume of the measuring channel (2e).

27. Device according to one of claims 5 to 26, wherein the outlet (1c) or a cannula (5b) placed on a cannula fitting (1b) or a flow reduction piece (6) has a smaller outlet cross-section than the cross-section of the measuring channel (2e).

28. Device according to one of claims 5 to 27, wherein the cross-section of the outlet (1c), the cross-section of the measuring channel (2e) and the volume of the buffer (2d) are dimensioned according to each particular application and the pressures required for it, such that there is at least partial ingress of the fluid (4a; 4b) into the measuring channel (2e) during handling.

## Revendications

1. Procédé pour le maniement contrôlable par pression d'un fluide (4a ; 4b), excepté des procédés pour le traitement chirurgical ou thérapeutique du corps humain ou animal et des procédés diagnostiques effectués au corps humain ou animal,
- le fluide (4a; 4b) se trouvant dans un réservoir (1a) de volume variable étant mis à disposition par une pression agissant sur le fluide (4a; 4b) au niveau d'un orifice de sortie (1c) avec cette pression,
- le réservoir (la) étant en communication avec un canal de section transversale prédéterminée,
- le canal étant en communication avec un tampon (2d), le tampon (2d) ayant un volume,
- le canal et le tampon (2d) étant remplis avec un milieu compressible,
**caractérisé en ce que** selon la pression effectuée, la section transversale du canal formant un canal de mesure (2e) et la grandeur prédéterminée du volume du tampon (2d) le fluide (4a ; 4b) pénètre au moins partiellement dans le canal de mesure (2e), durant le maniement dudit fluide (4a; 4b) la position de la couche limite (8) entre le fluide (4a; 4b) et le milieu compressible dans le canal de mesure (2e) étant observée ou mesurée et que le maniement comprend la génération d'une certaine pression dans le réservoir (1a) et le mouvement d'une canule (5b) dans un corps (9), ladite canule (5b) étant reliée à l'orifice de sortie, pour trouver et pour identifier dans le corps (9) une région (10) qui présente une pression qui diffère de la certaine pression générée en observant la position de la couche limite (8).

2. Procédé selon la revendication 1, le maniement étant l'injection contrôlable par pression d'un fluide (4a; 4b) par l'orifice de sortie (1c) hors du réservoir (1a) dans un corps (9).

3. Procédé selon la revendication 1 ou 2, la pression agissant sur le fluide (4a ; 4b) étant générée par un piston (2a) pouvant être poussé dans le réservoir (1a), ledit piston étant amené de façon étanche (2c) contre les parois intérieures du réservoir (1a).

4. Procédé selon la revendication 1, la pression agissant sur le fluide (4a ; 4b) étant ajustée et/ou réglée en fonction de la position de la couche limite (8).

5. Dispositif pour le maniement contrôlable par pression d'un fluide (4a ; 4b) avec
- un réservoir (1a) ayant un volume variable pour recevoir le fluide (4a ; 4b),
- un moyen de génération de pression (2a) pour la génération d'une pression agissant sur le fluide (4a ; 4b) ;
- un orifice de sortie (1c) pour l'entrée et/ou la sortie du fluide (4a ; 4b),
- un canal de section transversale prédéterminée étant en communication avec le réservoir (1a),
**caractérisé en ce que** le dispositif a un tampon (2d) avec un volume prédéterminé qui communique avec le canal comme canal de mesure (2e) et qui au moment du maniement contrôTable par pression est limité jusqu'à une hauteur possible d'un fluide dans la section transversale du canal de mesure par des parois fermes, le canal de mesure (2e) et le tampon (2d) étant remplis avec un milieu compressible.

6. Dispositif selon la revendication 5, le moyen de génération de pression étant un piston (2a) pouvant être poussé dans le réservoir (1a), ledit piston (1a) étant rendu étanche par rapport à la paroi intérieure du réservoir (1a) par des moyens d'étanchéité (2c).

7. Dispositif selon la revendication 6, le canal de mesure (2e) étant agencé dans le piston (2a) et débouchant avec une première ouverture (2f) dans l'espace pour le fluide (4a ; 4b) formé par le réservoir (1a) et le piston (2a).

8. Dispositif selon la revendication 6 ou 7, le canal de mesure (2e) débouchant avec une deuxième ouverture (2g) dans un tampon (2d).

9. Dispositif selon la revendication 6, 7 ou 8, le tampon (2d) étant agencé dans le piston (2a).

10. Dispositif selon l'une des revendications 6 à 9, le piston (2a) et/ou le canal de mesure (2e) présentant un moyen de mesure (2k) pour mesurer la pression du fluide (4a ; 4b).

11. Dispositif selon l'une des revendications 7 à 10, les parois du réservoir (1a), du piston (2a) et du canal de mesure (2e) étant transparentes et une de ces parois, de préférence celle du canal de mesure (2e), présentant une échelle graduée (2k) pour déterminer la position de la couche limite (8) entre le fluide (4a ; 4b) et le milieu compressible dans le canal de mesure (2e).

12. Dispositif selon l'une des revendications 6 à 11, le milieu compressible et le fluide (4a ; 4b) se trouvant dans le réservoir (1a) étant séparés l'un de l'autre par un moyen de séparation (3) qui ne se mélange pas avec le fluide (4a ; 4b) et le milieu compressible, par exemple par un bouchon élastique ou une goutte d'huile.

13. Dispositif selon la revendication 12, le moyen de séparation (3) étant déplaçable de façon étanche dans le canal de mesure (2e), mais avec une résistance négligeable par rapport aux autres forces se manifestant.

14. Dispositif selon l'une des revendications 6 à 13, le tampon (2d) étant agencé à l'intérieur du piston (2a) et le canal de mesure (2e) étant agencé de manière centrée à l'intérieur du tampon (2d).

15. Dispositif selon l'une des revendications 6 à 13, le canal de mesure (2e) étant disposé de manière centrée dans le piston (2a).

16. Dispositif selon la revendication 15, le canal de mesure (2e) se trouvant près d'une paroi extérieure du piston (2a) ou dans la paroi extérieure du piston (2a).

17. Dispositif selon l'une des revendications 6 à 16, la grandeur du volume du tampon (2d) étant variable.

18. Dispositif selon la revendication 17, le volume du milieu compressible se trouvant dans le tampon (2d) du piston (2a) pouvant être défini par une pièce obturatrice (2h ; 2i ; 2j) agencée dans le piston (2a).

19. Dispositif selon la revendication 18, la pièce obturatrice (2h ; 2i ; 2j) étant mobile et le volume du tampon (2d) pouvant être ajusté par ce moyen.

20. Dispositif selon l'une des revendications 14 à 16, le milieu compressible se trouvant dans le canal de mesure (2e) étant séparable du milieu compressible se trouvant dans le tampon (2d) du piston (2a) par une pièce obturatrice (2i).

21. Dispositif selon l'une des revendications 5 à 20, le tampon (2d) présentant une ouverture de communication avec l'atmosphère ambiante pouvant être fermée.

22. Dispositif selon l'une des revendications 5 à 21, le milieu compressible étant un gaz, notamment de l'air.

23. Dispositif selon l'une des revendications 5 à 22, le dispositif étant construit sensiblement de façon cylindrique.

24. Dispositif selon l'une des revendications 5 à 23, le dispositif étant une seringue, notamment pour des applications médicales.

25. Dispositif selon l'une der revendications 5 à 24, des moyens de frottement étant présents (2c) qui retiennent par de la force venant de l'extérieur, même dans le cas de soulagement de cette force, relativement au réservoir (1a) le piston (2a) dans chaque position ajustée.

26. Dispositif selon l'une des revendications 5 à 25, le volume du tampon (2d) étant au moins 10 fois, de préférence 100 fois plus grand que le volume du canal de mesure (2e).

27. Dispositif selon l'une des revendications 5 à 26, l'orifice de sortie (1c) ou une canule (5b) posée sur un ensemble de canules (1b) ou une pièce de réduction de débit (6) présente une section transversale d'orifice de sortie plus petite que la section transversale du canal de mesure (2e).

28. Dispositif selon l'une des revendications 5 à 27, dans le cas duquel selon le cas d'application et selon les pressions nécessaires la section transversale de l'orifice de sortie (1c), la section transversale du canal de mesure (2e) et le volume du tampon (2d) sont dimensionnés de telle façon que lors du maniement le fluide (4a ; 4b) pénètre au moins partiellement dans le canal de mesure (2e).
